**Europäisches Patentamt**

**European Patent Office**

**Office européen des brevets**

(19)

(11) Veröffentlichungsnummer: **0 363 770**

**A1**

## (12) EUROPÄISCHE PATENTANMELDUNG

(21) Anmeldenummer: **89118186.9**

(22) Anmeldetag: **30.09.89**

(51) Int. Cl.⁵: **A61M 5/00 , A61M 39/00**

(30) Priorität: **03.10.88 DE 8812460 U**

(43) Veröffentlichungstag der Anmeldung:
**18.04.90 Patentblatt 90/16**

(84) Benannte Vertragsstaaten:
**AT BE CH DE ES FR GB GR IT LI NL SE**

(71) Anmelder: **Schiwa GmbH**
**Postfach 11 80**
**D-4519 Glandorf(DE)**

(72) Erfinder: **Rycyk, Manfred**
**Lortzingstrasse 14**
**D-4516 Bissendorf(DE)**
Erfinder: **Recker, Antonius**
**Sudendorf 32**
**D-4519 Glandorf(DE)**
Erfinder: **Wesseler, Matthias**
**Uphoefener Feld 16**
**D-4517 Hilter 2(DE)**
Erfinder: **Falk, Guenter**
**Ottoschacht 3**
**D-4504 Georgsmarienhuette(DE)**

(74) Vertreter: **Höller, Klaus, Dr. et al**
**BASF Aktiengesellschaft Carl-Bosch-Strasse 38**
**D-6700 Ludwigshafen(DE)**

(54) **Konnektor für einen Behälter für pharmazeutische Lösungen.**

(57) Der Konnektor dient zur Verbindung zweier Behälter (3, 4) und besteht im wesentlichen aus 2 Teilen: einem rohrartigen Kopplungsteil (1) und einem dieses abschließenden Absperrorgan (5). Das Kopplungsteil (1) setzt sich aus einem hohlen Einstechdorn (6) und einem sich daran anschließenden, in ein schlauchförmiges Anschlußteil (2) eines Behälters (3) einsteckbaren, hohlen Teil (8) zusammen, das durch das von außen lösbare Absperrorgan (5) abgeschlossen ist.

EP 0 363 770 A1

## Konnektor für einen Behälter für pharmazeutische Lösungen

Die Erfindung betrifft einen Konnektor für einen Behälter für pharmazeutische Lösungen, der mit einem schlauchförmigen Anschlußteil versehen ist, bestehend aus einem in das Anschlußteil einsteckbaren, rohrartigen Kopplungsteil und einem das Kopplungsteil abschließenden Absperrorgan.

Zur Überleitung pharmazeutischer Lösungen von einem Behälter in einen anderen, beispielsweise von einer Flasche mit Inhaltsstoffen in einen Infusionsbeutel, muß sichergestellt sein, daß die Verbindung der beiden Behälter kontaminationsfrei hergestellt werden kann.

Dies erfolgte bisher dadurch, daß mittels einer Spritze mit aufgesetzter Einmalkanüle beispielsweise physiologische Kochsalzlösung aus einer Glasflasche entnommen und in einen Behälter mit Trockensubstanz gegeben wurde. Das dann darin vermischte Medium wurde anschließend mittels Infusionsgerät zum Patienten übergeleitet.

Als nachteilig anzusehen ist dabei, daß einerseits dieses Verfahren sehr langwierig ist, da dabei darauf geachtet werden muß, daß bei der Zugabe des flüssigen Mediums in den Trockensubstanzbehälter kein Überdruck entsteht, andererseits sich beim Entfernen der Kanüle aus dem Stopfen des Behälters u.U. Aerosole entstehen können. Beim Umgang mit hochtoxischen Substanzen, beispielsweise Cytostatika, führen Kontaminationen zu gefährlichen Erkrankungen.

Demgemäß bestand die Aufgabe, einen Konnektor für einen Behälter zu entwickeln, der eine kontaminationsfreie Verbindung mit einem anderen Behälter ermöglicht, wobei die Verbindung eine Sperre besitzen soll.

Die Aufgabe wurde durch einen Konnektor der eingangs geschilderten Art gelöst, bei dem gemäß der Erfindung das Kopplungsteil aus einem mit einem rohrartigen Einsteckteil verbundenen hohlen Einstechdorn besteht und sich innerhalb des Anschlußteils an das Einsteckteil ein Absperrorgan anschließt, welches sich von außen in eine Öffnungsstellung bringen läßt.

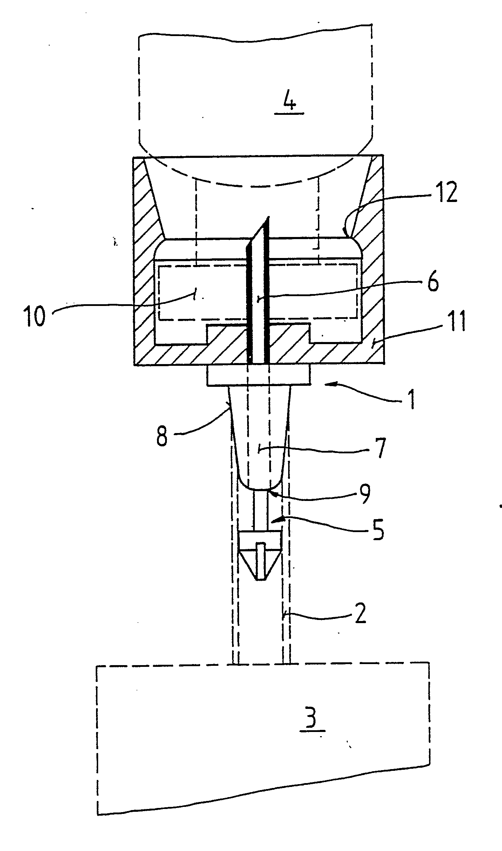

In der Zeichnung ist ein Ausführungsbeispiel des Konnektors schematisch dargestellt, anhand derer die Erfindung nachfolgend erläutert ist.

Der Konnektor besteht im wesentlichen aus 2 Teilen: einem rohrartigen Kopplungsteil 1, welches einerseits in ein schlauchförmiges Anschlußteil 2 des Behälters 3 dichtend einsteckbar ist und andererseits mit einem anderen ·Behälter 4 verbunden werden kann, und einem das Kopplungsteil abschließenden Absperrorgan 5, das an der dem Behälter 3 zugewandten Seite des Kopplungsteils lösbar angebracht ist.

Das Kopplungsteil 1 besteht aus einem hohlen Einstechdorn 6 und einem damit verbundenen, einen Durchflußkanal 7 aufweisenden, stopfenartigen Einsteckteil 8, das durch das Absperrorgan 5 behälterseitig abgeschlossen ist. Als Absperrorgan dient vorteilhafterweise ein an sich bekannter Abbrechverschluß, der einstückig mit dem Einsteckteil an einer zum Abbrechen vorgesehenen Schwächungszone 9 den Kanal 7 abschließt.

Der Konnektor wird mit dem Einsteckteil 8 in das Anschlußteil 2 des Behälters 3, z.B. in den Anschlußschlauch eines Infusionsbeutels, eingesetzt, während der andere Behälter 4, z.B. eine Flasche mit Inhaltsstoffen, mit seinem gummielastischen Stopfen 10 auf den Einstechdorn 6 aufzustecken ist. Nach dem Abbrechen des Verschlusses 5 ist der Überleitungsweg vom Behälter 4 zum Behälter 3 frei.

Es ist vorteilhaft, das Kopplungsteil 1 mit den Einstechdorn 6 umgebenden Stegen oder einem Aufnahmezylinder 11 zur Führung und Halterung des Behälterverschlußteils 10 zu versehen. Dabei hat es sich als zweckmäßig erwiesen, die Stege bzw. den Zylinder mit Vorsprüngen 12, beim Zylinder auch mit einem umlaufenden Vorsprung, auszustatten, um ein Arretieren des aufgesetzten Behälters 4 zu ermöglichen. Dadurch soll gewährleistet sein, daß der Behälter 4 für den Anwender unlösbar mit dem Behälter 3 verbunden ist.

## Ansprüche

1. Konnektor für einen Behälter für pharmazeutische Lösungen, der mit einem schlauchförmigen Anschlußteil versehen ist, bestehend aus einem in das Anschlußteil (2) einsteckbaren, rohrartigen Kopplungsteil und einem das Kopplungsteil (1) abschließenden Absperrorgan (5), dadurch gekennzeichnet, daß das Kopplungsteil (1) aus einem mit einem rohrartigen Einsteckteil (8) verbundenen hohlen Einstechdorn (6) besteht und sich innerhalb des Anschlußteils (2) an das Einsteckteil ein Absperrorgan (5) anschließt, welches sich von außen in eine Öffnungsstellung bringen läßt.

2. Konnektor nach Anspruch 1, dadurch gekennzeichnet, daß das Absperrorgan durch einen Abbrechverschluß (5,9) gebildet ist.

3. Konnektor nach Anspruch 1 und 2, dadurch gekennzeichnet, daß das Kopplungsteil (1) mit Führungen (11) zur Aufnahme eines einen gummielastischen Stopfen (10) aufweisenden Verschlußteils versehen ist, die den Einstechdorn (6) umgeben.

4. Konnektor nach Anspruch 3, dadurch gekennzeichnet, daß die Führungen (11) Arretierungsvorsprünge (12) für den Behälter (4) aufweisen.

5. Konnektor nach Anspruch 4, dadurch gekennzeichnet, daß die Arretierungsvorsprünge (12) so ausgebildet sind, daß nach Konnektion des Stopfens (10) der Behälter (4) unlösbar mit dem Konnektor verbunden ist.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.X5 |
|---|---|---|---|
| Y | US - A - 3 123 072 (D. BELLAMY) * Fig. 10,11; Spalte 5, Zeile 54 - Spalte 6, Zeile 10 * -- | 1-5 | A 61 M 5/00 A 61 M 39/00 |
| Y | US - A - 4 675 020 (CH.J. McPHEE) * Fig. 3,4; Spalte 2, Zeile 64 - Spalte 3, Zeile 56; Spalte 4, Zeile 60 - Spalte 5, Zeile 3; Spalte 5, Zeilen 18-30; Spalte 6, Zeilen 19-39 * -- | 1-5 | |
| A | EP - A1 - 0 028 198 (LAB. AGUETTANT) * Fig. 5; Seite 5, Zeilen 22-25; Seite 6, Zeilen 21-25 * -- | 1,2 | |
| A | US - A - 4 181 140 (E.L. BAYHAM et al.) * Gesamt * ---- | 1,2 | **RECHERCHIERTE SACHGEBIETE (Int. Cl.X5** A 61 M 5/00 A 61 M 39/00 |

Der vorliegende Recherchenbericht wurde für alle Patentansprüche erstellt.

| Recherchenort | Abschlußdatum der Recherche | Prüfer |
|---|---|---|
| WIEN | 26-01-1990 | LUDWIG |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82